# EUROPEAN PATENT SPECIFICATION

(11) **EP 3 602 038 B1**
(45) Date of publication and mention of the grant of the patent: **12.02.2025**
(21) Application number: 18717721.7
(22) Date of filing: 28.03.2018
(51) Int. Cl.: G01N 33/08

(54) **INSPECTION DEVICE AND METHOD FOR INSPECTING EGGS FOR IRREGULARITIES**
INSPEKTIONSVORRICHTUNG UND -VERFAHREN ZUR INSPEKTION VON EIERN AUF UNREGELMÄSSIGKEITEN
DISPOSITIF ET PROCÉDÉ D'INSPECTION D'IRRÉGULARITÉS DANS DES OEUFS

(30) Priority: 28.03.2017 NL 2018590
(43) Date of publication of application: 05.02.2020
(73) Proprietor: Van de Ven Beheer B.V., 5521 DW Eersel (NL)
(72) Inventor: VAN DORST, Niels Franciscus Gerardus, 5611 CX Eindhoven (NL); STORTELDER, Paulus Johannus Hendricus Maria, 7122 CD Aalten (NL)
(74) Representative: Algemeen Octrooi- en Merkenbureau B.V.
(86) International application number: PCT/NL2018/050187
(87) International publication number: WO 2018/182407

(56) References cited:
- US-A- 2 150 375
- US-A- 4 671 652
- US-A- 5 745 228
- US-A- 5 898 488
- US-A1- 2009 201 323
- US-A1- 2011 141 455
- US-B2- 7 796 241

## Description

### Background of the invention

The present invention relates to an inspection device for inspecting eggs for irregularities. Several types of inspection devices are already known in the art. One of such known inspection device comprises a conveyer unit comprising egg receiving holders, wherein said receiving holders are situated such that they form a uniform grid of receiving holders, wherein said conveyer unit is arranged for conveying, in a conveying direction, eggs received in said receiving holders and for rotating said eggs in said receiving holders during said conveyance. It further comprises an image recording unit positioned, in use, above said conveyer unit and arranged for recording images of said eggs during said conveyance, wherein each image comprises eggs in a particular uniform grid of receiving holders. It further comprises an illumination unit positioned, in use, below said egg receiving holders and arranged for illuminating said eggs in said particular uniform grid of receiving holders by means of lasers. It further comprises a data processing unit arranged for determining whether eggs have irregularities based on said recorded images.

One of the drawbacks of these known inspection devices is that they require relatively a lot of processing, by the data processing unit, to determine whether eggs have irregularities.

US 5,898,488A provides a means for candling eggs. The apparatus includes a conveying means removing trays of eggs from an infeed station to an outfeed station, candling performed at the first station by a video camera.

US 20090201323 A1 discloses a system applicable to an installation for candling eggs, to determine the presence of fertilized eggs in the cells of the egg crate grid moving on a conveyor.

US 20110141455 A1 discloses egg candling method for determining a state of eggs between the 13th and the 17th day of incubation.

### Summary of the invention

It is an object of the present invention to provide for an inspection device that is able to determine whether eggs have irregularities using less processing compared to the inspection device in accordance with the prior art.

The object is achieved, in a first aspect, by an inspection device for inspecting eggs for irregularities, said inspection device comprising:
- a conveyer unit comprising egg receiving holders, wherein said receiving holders are situated such that they form a uniform grid of receiving holders, wherein said conveyer unit is arranged for conveying, in a conveying direction, eggs received in said receiving holders and for rotating said eggs in said receiving holders during said conveyance,
- an image recording unit positioned, in use, above said conveyer unit and arranged for recording images of said eggs during said conveyance, wherein each image comprises eggs in a particular uniform grid of receiving holders;
- an illumination unit positioned, in use, below said egg receiving holders and arranged for illuminating said eggs in said particular uniform grid of receiving holders, wherein said illumination unit comprises a plurality of light sources each arranged to illuminate one egg in said particular uniform grid of receiving holders,
- a data processing unit arranged for determining whether eggs have irregularities based on said recorded images.

The inspection device is characterized in that the illumination unit is arranged for illuminating said eggs in said particular uniform grid of receiving holders such that adjacent eggs, seen in said conveying direction, in said particular uniform grid are illuminated with different intensity; or
said illumination unit is arranged for illuminating eggs in said particular uniform grid of receiving holders such that adjacent eggs, seen in said conveying directing, in said particular uniform grid are illuminated alternately..

It was an insight of the inventors that the quality of the images recorded by the image recording unit is improved in case the adjacent eggs, seen in the conveying direction, in the particular uniform grid are illuminated with different intensity.

The inventors have found that the scattering of the light from a particular egg to the neighbouring eggs, i.e. the adjacent eggs, is one of the reasons why the quality of the images recorded by the image recording unit of the prior art was relatively low. This required a lot of images to be taken per egg and/or more extensive (pre-)processing of the images to still be able to determine whether eggs have irregularities or not.

The inspection device of the present invention has a different working principle. Neighbouring eggs, i.e. adjacent eggs, seen in the conveying direction, are illuminated with different intensity. For example a particular egg may be illuminated fully, and the adjacent eggs, seen in the conveying direction, may not be illuminated at all. The image recording unit will then record an image, wherein the image mainly reflects that particular egg. That is, the image mainly reflects the eggs that have been fully illuminated, and the data processing unit can use such a particular image for the fully illuminated eggs only. The recorded images are thus of a higher quality. The eggs that have not been fully illuminated are not considered, by the data processing unit, at that time. As such, scattering of light from one egg to a neighbouring, i.e. adjacent, egg, becomes less of an issue.

In a next image of the image recording unit, the illumination pattern may differ. That is, the eggs that were fully illuminated in the previous image are this time not illuminated. The eggs that were not illuminated previously, are now fully illuminated by the illumination unit. Again, the adjacent eggs are thus illuminated with different intensity. The data processing unit can use this next image for determining whether the freshly, newly, illuminated eggs have irregularities.

The above thus entails that the images recorded by the image recording unit are of a higher quality. The result hereof is that fewer images are, and/or less extensive (pre-)processing of the images is, required for determining whether eggs have irregularities or not.

In accordance with the present disclosure, an irregularity may be eggshell damage, a thin eggshell, non-uniform eggshell thickness (e.g. a calcium deposit), a stain on the eggshell (e.g. a manure stain) and/or abnormality in shape. The irregularity is directed to the outer surface of the egg, i.e. the eggshell.

In accordance with the present disclosure, the eggs are rotated, by the conveyer unit, in the receiving holders during the conveyance. This allows for the image recording unit to record multiple images of the egg, wherein, for each image, the egg is rotated with respect to the previous images. This ensures that the recorded images reflect the eggs from all kinds of orientations such that the circumferences of all the eggs have been covered.

The image recording unit comprises, for example, one or multiple camera's for recording images of the eggs that are being conveyed below the camera's. The eggs are conveyed, by the conveyer unit, in a uniform grid. As such, each image of the camera will reflect multiple eggs present in the uniform grid. The uniform grid is, for example, formed by columns and rows of eggs, wherein the direction of a row is parallel to the conveying direction, and wherein the direction of a column is transverse to the conveying direction. The image recording unit may, for example, be arranged to record images covering eggs in five to seven eggs per column, and four eggs per row. As such, images are recorded covering about twenty to twenty-eight eggs at the same time.

The illumination unit is arranged to illuminate the eggs in the particular uniform grid of receiving holders. The illumination unit is positioned, in use, below the egg receiving holders. This means that the illumination unit will provide for an illumination which is directed upwards. The illumination unit and the image recording unit should be synchronized to a certain extent. That is, an image should be recorded during illumination of the eggs.

Preferably, the illumination unit illuminates the eggs using a red colour. The inventors have found that the colour red is beneficial as light having such a wavelength is least absorbed by the eggs. The result is that the illuminated unit may operate with reduced power consumption. The data processing unit is, for example, a computer present at, or in the vicinity, of the image recording unit. In another example, the data processing unit is a cloud based platform or a distributed based computing unit.

It is noted that the illumination unit may comprise a plurality of light sources each arranged to illuminate one egg in said particular uniform grid of receiving holders. Each light source will thus illuminate a single egg in said uniform grid of receiving holders.

In an example, the particular uniform grid resembles a checkerboard, wherein a first group of grid positions in said particular grid resembles white fields of said checkerboard and wherein a second group of grid positions in said particular grid resembles black fields of said checkerboard, wherein said illumination unit is further arranged to:
- alternately illuminate eggs in said first group of grid positions and eggs in said second group of grid positions.

The advantage of the example disclosed above is that the illumination unit has to provide for an illumination only twice to cover all the eggs in the uniform grid. That is, in a first step, all eggs present in the first group of grid positions are illuminated and, in second step, all eggs present in the second group of grid positions are illuminated.

First, the eggs in the first group are illuminated, by the illumination unit, and the eggs in the second group of grid positions are not illuminated. An image is then recorded, by the image recording unit. The recorded image is then used for determining whether eggs have irregularities, but only for those eggs that have been illuminated by the illumination unit, i.e. the eggs in the first group of grid positions.

Second, the eggs in the second group are illuminated, by the illumination unit, and the eggs in the first group of grid positions are not illuminated. Again, an image is recorded, by the image recording unit. This particular recorded image is then used for determining whether eggs have irregularities, but only for those eggs that have been illuminated by the illumination unit, i.e. the eggs in the second group of grid positions.

The time difference between the illuminating of the eggs in first group of grid positions and the eggs in the second group of grid positions is relatively small, for example a couple of microseconds, milliseconds or couple of hundreds of seconds.

The issue of scattering of the light from a particular egg to the neighbouring eggs, seen in the conveying direction, is reduced by using the checkerboard example as disclosed above.

It is noted that a regular checkerboard comprises sixty four fields, i.e. thirty two black coloured field and thirty two white coloured fields. This present example is not limited to the amount of fields in a checkerboard. Typically, a checkerboard principle of about twenty to twenty eight fields are used, i.e. about ten black coloured fields and ten white coloured fields up to fourteen black coloured fields and fourteen white coloured fields.

In a further example, said conveyer unit, said image recording unit and said illumination unit are mutually arranged such that each egg is illuminated between four times and ten times, preferably between four times and eight times, even more preferably four times, and such that each time said egg is illuminated an image is recorded, and such that for each recorded image said egg is rotated between 80 degrees and 120 degrees, preferably between 80 and 100 degrees, even more preferably around 90 degrees.

The advantage of the example provided above is that only four images are required for covering the whole egg. The recording angle of the image recording unit, i.e. the viewing angle of the image recording unit, is typically about 120°. This means that about 120° of a particular egg is recorded per image. The particular egg is then rotated, for example about its longitudinal axis, for example between 80 degrees and 120 degrees. The next image of that particular egg then covers again about 120° of that particular egg. Of course, there can be overlap with the previous image of that particular egg, but that overlap is ignored or used in both images to more accurately determine whether there are irregularities in the particular egg.

The rotating of the eggs in the receiving holders is, typically, a mechanical process. As such, the speed at which the eggs rotate is the same for each egg. A large egg has a larger circumference compared to a small egg. This means that it takes more time to fully rotate a relatively large egg compared to a relatively small egg.

The conveyer unit, i.e. the position/orientation of the rollers on which the eggs reside, the image recording unit and the illumination unit are then arranged in such a way, that relatively large eggs are rotated about 80 degrees before the next image is recorded and that relatively small eggs are rotated about 120 degrees before the next image is recorded.

In a further example, the inspection device further comprises:
- an egg detection unit arranged for detecting whether eggs are received in said receiving holders,
and wherein said illumination unit is further arranged to refrain from illuminating a receiving holder in case said egg detection unit has not detected a received egg in said receiving holder.

The advantage of this example is that the image recording unit is not subjected to an overexposure in case one or more eggs are not received in the receiving holders. The inventors have found that the illumination unit may cause an overexposure at the image recording unit in case no egg is present in a receiving holder. The provided illumination is then not blocked by an egg, and the provided illumination is then in line-of-sight with the image recording unit. This is a disadvantage as this could degrade the quality of the images recorded by the image recording unit. As such, illumination unit will make sure that the receiving holders that do not have received an egg, are not illuminated.

In yet another example, the inspection device further comprises:
- an egg size detection unit arranged for detecting sizes of said eggs in said receiving holders,
and wherein said illumination unit is further arranged for determining said intensity of said illumination based on said detected sizes of said eggs.

The inventors have found that the quality of the images are further improved if the intensity of the illumination of the eggs is tuned to the size of the eggs. As such, an egg size detection unit is provided for detecting the size of the eggs in the receiving holders. The egg size detection unit is in communication with the illumination unit directly or indirectly. The illumination unit will then determine the required intensity of the illumination of each of the eggs separately based on the detected sized of each of the eggs.

For example, the intensity of the illumination should, preferably, be different for different sizes of eggs. As such, a relatively large egg may, for example, be illuminated more or less intensely compared to a relatively small egg.

In a practical example, the illumination unit is arranged to
- illuminate eggs in five rows of said uniform grid, wherein a direction of a row is parallel to said conveying direction, and eggs in four columns of said uniform grid, wherein a direction of a column is transverse to said conveying direction, or to
- illuminate eggs in six or seven rows of said uniform grid and eggs in four columns of said uniform grid.

In another example, the illumination unit comprises a plurality of Light Emitting Diode's, LED's, wherein each LED is arranged to illuminate an egg in a grid position of said particular uniform grid.

The inventors have found that it is beneficial if the illumination unit is able to control the illumination of each of the grid positions of the particular uniform grid separately. This may be achieved using a plurality of LED's, wherein each LED is arranged to illuminate an egg in a particular grid of the uniform grid. It is noted that, preferably, red coloured LED's are used as the colour red is least absorbed by the eggs themselves.

In an more detailed example hereof, each of said plurality of LEDs is provided with direction means, for example a lens and/or a lighting shaft, for directing illumination towards said corresponding egg in said grid position of said particular uniform grid.

The advantage of using lenses and/or lighting shafts is that the light illuminated by a particular LED is directed to its corresponding egg in the corresponding grid position only. As such, the stray light from that particular LED is reduced. Stray light is defined as the light that originates from a particular LED and illuminates a grid position which does not correspond to the grid positions of the particular LED.

The expressions, i.e. the wording, of the different aspects comprised by the method and devices according to the present disclosure should not be taken literally. The wording of the aspects is merely chosen to accurately express the rationale behind the actual functioning of the aspects.

In accordance with the present disclosure, different aspects applicable to the above mentioned examples of the devices, including the advantages thereof, correspond to the aspects which are applicable to the method in accordance with the examples provided below.

In a second aspect, there is provided a method for determining whether eggs have irregularities using an inspection device according to any of the previous claims, wherein said method comprises the steps of:
- conveying, by said conveyer unit, eggs received in said receiving holders, and rotating said eggs in said receiving holders during said conveyance,
- recording, by said image recording unit, images of said eggs during said conveyance, wherein each image comprises eggs in a particular uniform grid of receiving holders;
- illuminating, by said illumination unit, said eggs in said particular uniform grid of receiving holders, and
- determining, by said data processing unit, whether eggs have irregularities based on said recorded images,
characterized in that said step of illuminating further comprises:
illuminating, by said illumination unit, said eggs in said particular uniform grid of receiving holders such that adjacent eggs, seen in said conveying direction, in said particular uniform grid are illuminated with different intensity.

The advantage of this example is that the problem of scattering of light from a particular egg to its neighbouring egg is reduced. This is especially the case if the different intensity is such that a particular egg is illuminated fully, and the adjacent egg is not illuminated at all. In this case, each of the recorded images are only used for the eggs that have been illuminated.

In an example, the particular uniform grid resembles a checkerboard, wherein a first group of grid positions in said particular grid resembles white fields of said checkerboard and wherein a second group of grid positions in said particular grid resembles black fields of said checkerboard, wherein said step of illuminating further comprises:
- alternately illuminating eggs in said first group of grid positions and eggs in said second group of grid positions.

In a further example, the conveyer unit, said image recording unit and said illumination unit are mutually arranged such that each egg is illuminated four times, and such that each time said egg is illuminated an image is recorded, and such that for each recorded image said egg is rotated between 80 degrees and 120 degrees, preferably between 80 and 100 degrees, even more preferably around 90 degrees.

In yet another example, the method further comprises the steps of:
- detecting, by said egg detection unit, whether eggs are received in said receiving holders, and
- refraining, by said illumination unit, from illuminating a receiving holder in case said egg detection unit has not detected a received egg in said receiving holder.

In an example, the method further comprises the steps of:
- detecting, by said egg size detection unit, said sizes of said eggs in said receiving holders, and
- determining, by said illumination unit, said intensity of said illumination based on said detected sizes of said eggs.

In yet another example, the step of illuminating further comprises:
- illuminating, by said illumination unit, eggs in five rows of said uniform grid, wherein a direction of a row is parallel to said conveying direction, and eggs in four columns of said uniform grid, wherein a direction of a column is transverse to said conveying direction, or to
- illuminating, by said illumination unit, eggs in six or seven rows of said uniform grid and eggs in four columns of said uniform grid.

In a third aspect, the present disclosure provides for a computer program product, comprising a readable storage medium, comprising instructions which, when executed on at least one processor, cause the at least one processor to carry out the method according to any of the examples as provided above.

The above-mentioned and other features and advantages of the disclosure will be best understood from the following description referring to the attached drawings. In the drawings, like reference numerals denote identical parts or parts performing an identical or comparable function or operation.

### Brief description of the drawings

Figure 1 shows an inspection device in accordance with the present disclosure.
Figure 2 shows a part of a conveyer unit comprised by an inspection device in accordance with the present disclosure.
Figure 3 shows a part of a conveyer unit as well as an illumination unit comprised by an inspection device in accordance with the present disclosure.
Figure 4a and 4b show the checkerboard example in accordance with the present disclosure.

### Detailed description

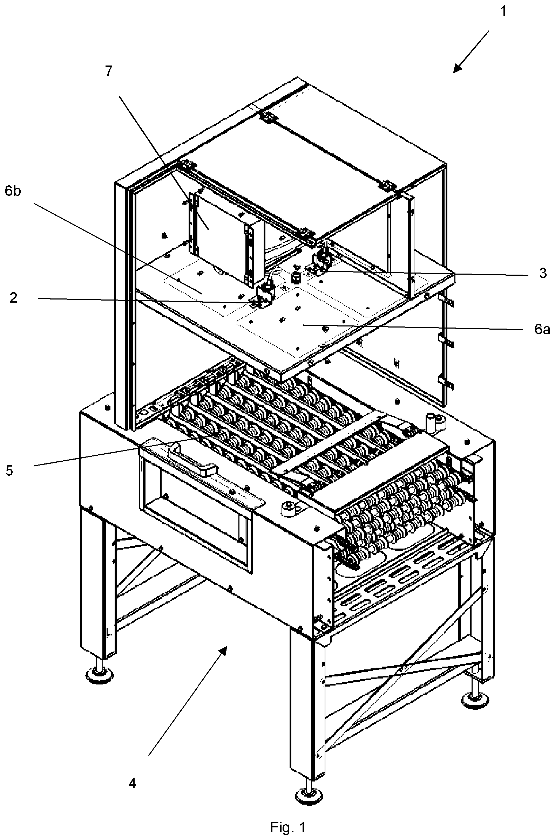

Figure 1 shows an inspection device 1 for inspecting eggs for irregularities. The inspection device 1 is thus used for determining whether eggs have irregularities. Each egg having an irregularity may subsequently be sorted out. The egg having the irregularity may be destroyed, thrown away or processed in any other manner.

Multiple types of irregularities may be checked for using the inspection device 1. A typical irregularity is an open crack in the egg. Open cracks in eggs may cause leaking of the eggs. This in turn may cause soiling of the inspection device and/or soiling of other eggs. It is therefore beneficial if eggs having an open crack are detected as soon as possible to prevent such things to happen. Other types of irregularities include calcium deposit, a manure stain and/or a manure sweep.

As mentioned above, different types of irregularities exist which may be detected b the device according to the present disclosure. For example, an irregularity may be eggshell damage, a thin eggshell, non-uniform eggshell thickness (e.g. a calcium deposit), a stain on the eggshell (e.g. a manure stain) and/or abnormality in shape.

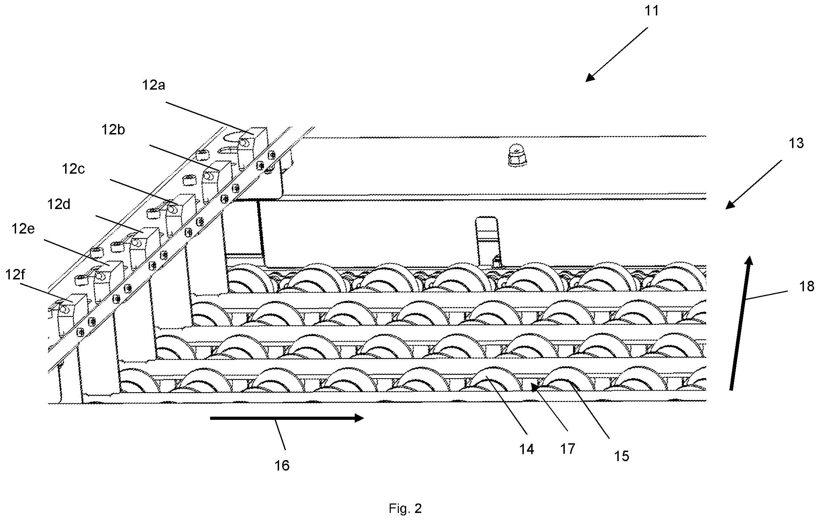

The inspection device 1 comprises a conveyer unit 5 for conveying, in a conveying direction, eggs receiving in receiving holders. The receiving holders and the conveying direction are explained in more detail with respect to figure 2. Further, the receiving holders are situated such that they form a uniform grid of receiving holders.

The eggs received in the receiving holders are rotated, for example around their longitudinal axis, during the conveyance.

An image recording unit 2, 3 is provided which is, in use, situated above the conveyer unit 5. The image recording unit 2, 3 comprises, in this particular example, two camera's. A first camera is indicated with reference numeral 2 and a second camera is indicated with reference numeral 3. The image recording unit 2, 3 is arranged for recording images of the eggs during the conveyance, wherein each image comprises eggs in a particular uniform grid of receiving holders. Each camera 2, 3 may cover its own part of the particular uniform grid.

A uniform grid is a grid which has a translation symmetry in each dimension. In this particular example the rows and the columns of the conveyer unit 5 are aligned with respect to each other. This is also explained in more detail with respect to figure 2.

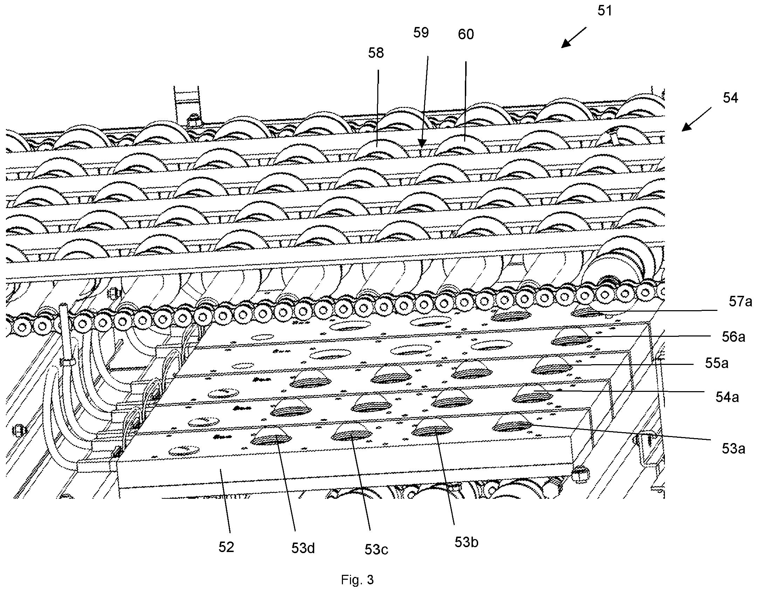

Further, an illumination unit 4 is provided which is positioned, in use, below the egg receiving holders and is arranged for illuminating the eggs in the particular uniform grid of receiving holders. The inspection device 1 is characterized in that the illumination unit 4 is arranged for illuminating the eggs in the particular uniform grid of receiving holders such that adjacent eggs, seen in the conveying direction, in the particular uniform grid are illuminated with different intensity. The illumination unit 4 is described in more detail with respect to figure 3.

It is noted that, typically, the eggs are also illuminated from a further, i.e. additional, illumination unit, which further illumination unit is positioned, in use, above the egg receiving holders. This further illumination unit, typically, provides white coloured, for example diffuse, illumination towards the eggs. The present disclosure does not describe this further illumination unit in detail.

Finally, a data processing unit 7 is comprised by the inspection device 1, which data processing unit 7 is arranged for determining whether eggs have irregularities base don the recorded images.

Figure 2 shows a part 11 of a conveyer unit 13 comprised by an inspection device in accordance with the present disclosure.

Here, the conveyer unit is indicated with reference numeral 13. The conveyer unit 13 is, for example, an endless conveyor provided with rollers 14, 15 extending transversely to the conveying direction 16. In this particular example, on two rollers have been identified, i.e. the rollers with reference numerals 14 and 15.

The rollers 14, 15 may be connected to each other using chains or anything alike. Typically, the rollers 14, 15 have the same size with respect to each other. The conveyor 13 has rows of a plurality rollers 14, 15, for example five rows or six rows or seven rows. The rows are oriented widthwise 18 next to each other.

The rollers 14, 15 are rotatably mounted which means that they can rotate about a rotating axis. In this particular situation, the rotating axis is parallel to the width direction 18.

The rollers 14, 15 generate receiving holders 17. Here, only one receiving holder 17 is stipulated for readability concerns. Eggs are thus effectively placed between rollers 14, 15. For example, one egg is placed between the rollers indicated with reference numerals 14 and 15, i.e. in the receiving holder indicated with reference numeral 17. Eggs placed in the receiving holders are rotated, around their longitudinal axis, by the rollers 14, 15. As such, adjacent rollers rotate in the same direction. For example, the roller indicated with reference numeral 15 rotates clockwise and the roller indicated with reference numeral 14 also rotates clockwise. In this case, the corresponding egg rotates anti-clockwise.

The receiving holders, and thus also the eggs received therein, are aligned with respect to each other. That is, the distances between the receiving holders in the conveying direction 16 are the same, and the distances between the receiving holders, in the width direction 18, are the same. As such, the receiving holders, and thus also the eggs received therein, are conveyed in the conveying direction 16 in a uniform grid.

In this particular example, an egg detection unit 12a-12f is provided which is arranged for detecting whether eggs are receiving in the receiving holders 17. The egg detection unit 12a-12f is further in communication, directly or indirectly, with the illumination unit, wherein the illumination unit is arranged to refrain from illuminating a receiving holder in case the egg detection unit 12a-12f has not detected a received egg in the receiving holder.

Figure 3 shows a part 51 of a conveyer unit 54 as well as an illumination unit 52 comprised by an inspection device in accordance with the present disclosure.

As mentioned above, the illumination unit is positioned, in use, below the egg receiving holders 59 and is arranged for illuminating the eggs in the particular uniform grid of receiving holders.

Only two rollers are indicated with reference numerals, being reference numerals 58 and 60. In between those rollers 58, 60, a receiving holder is formed for receiving an egg. Here, five rows of receiving holders are shown, wherein the receiving holders are aligned with each other.

The receiving holders, and thus also the eggs received therein, are illuminated using the illumination unit 52 which is positioned, in use, below the conveyer unit 54.

In this particular example, the illumination unit comprises twenty red coloured LED's 53a-53d, 54a, 55a, 56a, 57a, positioned in four columns (referenced to with reference numeral ending on an "a") and five rows. The rows are oriented parallel to the conveying direction. Here, the illumination unit 52 comprises five housings, wherein each housing corresponds to a single row. This is beneficial as this improves the scalability of the inspection device. The inspection device can be made tailored to a specific amount rows by simply adding, or removing, a housing.

The illumination unit 52 is configured such that it illuminates eggs in a particular uniform grid of receiving holders such that adjacent eggs, seen in the conveying direction, in the particular uniform grid are illuminated with different intensity.

Here, each of the LED's 53a-53d, 54a, 55a, 56a, 57a are arranged to illuminate a single receiving holder, i.e. egg in the receiving holder. In order to accomplish that, the LED 53a-53d, 54a, 55a, 56a, 57a may be provided with a lens for directing the illumination towards its corresponding receiving holder. As such, there is a one to one coupling between an LED and its corresponding receiving holder of the conveyer unit 54.

The LED's 53a-53d, 54a, 55a, 56a, 57a are then activated in such a way that adjacent LED's, and thus also adjacent receiving holders, are illuminated with different intensity. In this context, adjacent means at least the egg next to each other, seen from the conveying direction. The LED's 53a-53d, 54a, 55a, 56a, 57a may further be activated in such a way that adjacent LED's, seen in the width direction, are also illuminated with different intensity, in order to further improve the light scattering issue as explained above.

In this example, the particular uniform grid resembles a checkerboard, wherein a first group of grid positions in said particular grid resembles white fields of said checkerboard, and wherein a second group of grid positions in said particular grid resembles black fields of said checkerboard. The illumination unit 52 is then arranged to alternately illuminate the eggs in the first group of grid positions and eggs in the second group of grid positions.

First, the eggs in the first group of grid positions are illuminated and an image is recorded by the image recording unit. This particular image is used for those eggs that were illuminated, i.e. the eggs in the first group of grid positions.

Second, the eggs in the second group of grid positions are illuminated and an image is recorded by the image recording unit. This particular image is used for those eggs that were illuminated, i.e. the eggs in the second group of grid positions.

It is noted that, preferably, the time between the above mentioned two images is relatively small, i.e. a couple of microseconds, milliseconds or hundreds of seconds.

The illumination unit 52 is static. This means that the illumination unit 52 does not move during use. The conveyer unit 54 moves the eggs over the illumination unit 52. The eggs are rotated during the conveyance.

For example, consider the receiving holder which is currently associated with the LED indicated with reference numeral 53d. The egg is illuminated by the illumination unit 52 and an image is recorded. Then, that particular receiving holder moves forward, i.e. in the conveying direction, and is situated right above the next LED, i.e. the LED indicated with reference numeral 53. The egg is again illuminated by the illumination unit 52 and a next image is recorded. In this particular case, the egg is also rotated about its longitudinal axis, by its rollers. That is, the egg is rotated somewhere between 80 and 120 degrees, preferably around 90 degrees. As such, the second image covers a different part from that egg compared to the previous image.

Following the principle above, the egg is again illuminated by the LED indicated with reference numeral 53b and, subsequently, by the LED indicated with reference numeral 53a. Each time, an image is recorded, wherein the recorded images cover different parts of the egg. Finally, four images are recorded for a single egg.

It is noted that the image does not cover a single egg, but it covers the whole array of eggs. For example, in this particular situation, the image covers twenty eggs at the same time.

As an alternative to the illumination unit 52 as described above, a similar implementation may be construed. For example, the illumination unit 52 may also have eight columns, instead of four columns as in the example provided above. Then, the columns do not need to have an LED in each row, but could have an LED in each subsequent row. For example, the first column could have LED's in the first row, the third row and the fifth row. The second column could have LED's in the second row and the fourth row. The third column could resemble the first column. The fourth column could resemble the second column, etc. This example is a mechanical implementation which ensures that the eggs in the particular uniform grid of receiving holders are illuminated in such a way that adjacent eggs, seen in the conveying direction, in the particular uniform grid are illuminated with different intensity. In this particular example, the eggs may be rotated about 40 - 60 degrees, preferably 45 degrees, between recorded images.

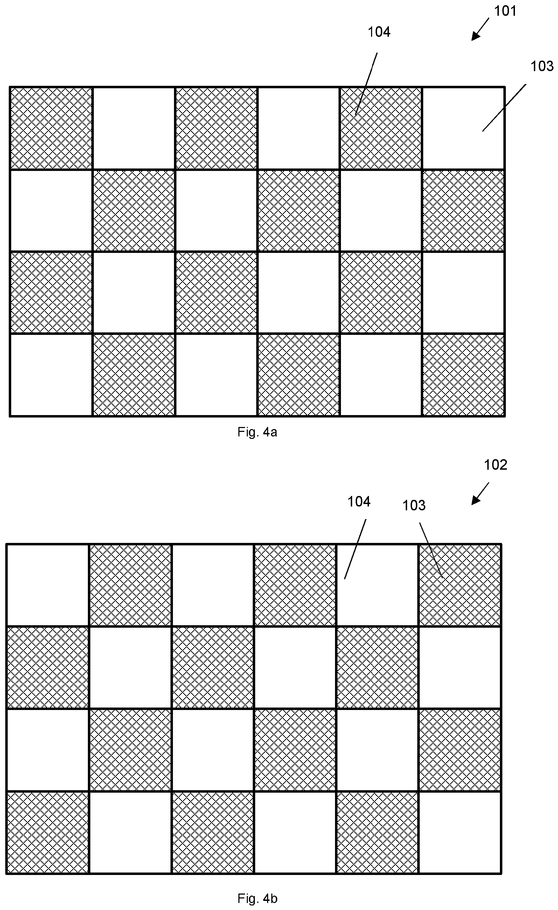

Figure 4a and 4b show the checkerboard example in accordance with the present disclosure. The checkerboard as disclosed here resembles the grid positions of the particular uniform grid in which the eggs are conveyed.

First, consider the checkboard of figure 4a, i.e. referenced to with reference numeral 101. In this particular example, the fields that are shaded, i.e. the one as indicated with reference numeral 104 in figure 4a, are illuminated. The fields that are not shaded, i.e. the one as indicated with reference numeral 103 in figure 4a, are not illuminated.

Each field of the checkboards as shown in figures 4a and 4b thus correspond to a particular receiving holder, wherein the receiving holders have received eggs. As shown, different eggs are illuminated in figure 4a as compared to figure 4b.

The recorded image corresponding to figure 4a can be used for assessing whether eggs have irregularities, but only for those eggs that are illuminated. These are the ones that correspond to the fields that are shaded. The recorded image corresponding to figure 4b can be used for assessing whether eggs have irregularities, but only for those eggs that are illuminated. These are the ones that correspond to the field that are shaded. The shaded field are, however, different from figure 4a and figure 4b. This ensures that all the eggs in the uniform grid are illuminated at least once.

One of the advantages of the inspection device in accordance with the present disclosure, is that the images recorded by the image recording unit are of relatively high quality. A reason is that light scattering, from a particular egg to its neighbouring egg, in the conveyance direction, is no longer a real issue. The neighbouring eggs are illuminated with different intensity, for example ON and OFF, such that the above mentioned scattering effect can be controlled. The result of images having a better quality is that less images are required to determine whether eggs have irregularities. As such, the data processing unit needs less images as an input for determining whether an egg has an irregularity, which decreases the total processing of the data processing unit.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope thereof.

## Claims

1. Inspection device (1) for inspecting eggs for irregularities, said inspection device (1) comprising:
- a conveyer unit (5, 13) comprising egg receiving holders (17), wherein said receiving holders (17) are situated such that they form a uniform grid of receiving holders, wherein said conveyer unit (5, 13) is arranged for conveying, in a conveying direction, eggs received in said receiving holders (17),
- an image recording unit (2, 3) positioned, in use, above said conveyer unit (5) and arranged for recording images of said eggs during said conveyance, wherein each image comprises eggs in a particular uniform grid of receiving holders (17);
- an illumination unit (4, 52) positioned, in use, below said egg receiving holders (17) and arranged for illuminating said eggs in said particular uniform grid of receiving holders (17), wherein said illumination (52) unit comprises a plurality of light sources (53a-53d, 54a, 55a, 56a, 57a) each arranged to illuminate one egg in said particular uniform grid of receiving holders (17, 59),
- a data processing unit (7) arranged for determining whether eggs have irregularities based on said recorded images;
**characterized in that**
wherein said conveyer unit is arranged for rotating said eggs in said receiving holders during said conveyance, allowing said image recording unit to record multiple images of each of said eggs, wherein for each image, the egg is rotated with respect to the previous images;
said illumination unit (4, 52) is arranged for illuminating said eggs in said particular uniform grid of receiving holders (17) such that adjacent eggs, seen in said conveying direction, in said particular uniform grid are illuminated with different intensity, or
said illumination unit (4, 52) is arranged for illuminating eggs in said particular uniform grid of receiving holders (17) such that adjacent eggs, seen in said conveying directing, in said particular uniform grid are illuminated alternately

2. Inspection device (1) according to claim 1, wherein said particular uniform grid resembles a checkerboard (101, 102), wherein a first group of grid positions in said particular grid resembles white fields of said checkerboard and wherein a second group of grid positions in said particular grid resembles black fields of said checkerboard, wherein said illumination unit (4, 52) is further arranged to:
- alternately illuminate eggs in said first group of grid positions and eggs in said second group of grid positions.

3. Inspection device (1) according to any of the previous claims, wherein said conveyer unit (5, 13), said image recording unit (2, 3) and said illumination unit (4, 52) are mutually arranged such that each egg is illuminated between four times and ten times, more preferably between four times and six times, even more preferably four times, and such that each time said egg is illuminated an image is recorded, and such that for each recorded image said egg is rotated between 80 degrees and 120 degrees, preferably between 80 and 100 degrees, even more preferably around 90 degrees.

4. Inspection device (1) according to any of the previous claims, wherein said inspection device further comprises:
- an egg detection unit (12a-12f) arranged for detecting whether eggs are received in said receiving holders,
and wherein said illumination unit (4, 52) is further arranged to refrain from illuminating a receiving holder in case said egg detection unit has not detected a received egg in said receiving holder.

5. Inspection device (1) according to any of the previous claims, wherein said inspection device (1) further comprises:
- an egg size detection unit arranged for detecting sizes of said eggs in said receiving holders,
and wherein said illumination unit (4, 52) is further arranged for determining said intensity of said illumination based on said detected sizes of said eggs.

6. Inspection device (1) according to any of the previous claims, wherein said illumination unit (4, 52) is arranged to
- illuminate eggs in five rows of said uniform grid, wherein a direction of a row is parallel to said conveying direction (16), and eggs in four columns of said uniform grid, wherein a direction of a column is transverse to said conveying direction (16), or to
- illuminate eggs in six or seven rows of said uniform grid and eggs in four columns of said uniform grid.

7. Inspection device (1) according to any of the previous claims, wherein said illumination unit comprises a plurality of Light Emitting Diode's, LED's (53a-53d, 54a, 55a, 56a, 57a), wherein each LED (53a-53d, 54a, 55a, 56a, 57a) is arranged to illuminate an egg in a grid position of said particular uniform grid.

8. Inspection device (1) according to claim 7, wherein each of said plurality of LEDs 53a-53d, 54a, 55a, 56a, 57a) is provided with directing means for directing illumination towards said corresponding egg in said grid position of said particular uniform grid.

9. A method for determining whether eggs have irregularities using an inspection device (1) according to any of the previous claims, wherein said method comprises the steps of:
- conveying, by said conveyer unit (5, 13), eggs received in said receiving holders, and,
- recording, by said image recording unit(2, 3), images of said eggs during said conveyance, wherein each image comprises eggs in a particular uniform grid of receiving holders;
- illuminating, by said illumination unit (4, 52), said eggs in said particular uniform grid of receiving holders, and
- determining, by said data processing unit (7), whether eggs have irregularities based on said recorded images,
**characterized in that** further comprising the step of:
rotating, by said conveyer unit (5, 13), said eggs in said receiving holders during said conveyance;
said step of recording further comprising:
recording, by said image recording unit(2, 3), multiple images of each of said eggs, wherein for each image, the egg is rotated with respect to the previous images;
said step of illuminating further comprises: illuminating, by said illumination unit (4, 52), said eggs in said particular uniform grid of receiving holders such that adjacent eggs, seen in said conveying direction, in said particular uniform grid are illuminated with different intensity; or
illuminating, by said illumination unit (4, 52), said eggs in said particular uniform grid of receiving holders such that adjacent eggs, seen in said conveying directing, in said particular uniform grid are illuminated alternately.

10. Method according to claim 9, wherein said particular uniform grid resembles a checkerboard (101, 102), wherein a first group of grid positions in said particular grid resembles white fields of said checkerboard and wherein a second group of grid positions in said particular grid resembles black fields of said checkerboard, wherein said step of illuminating further comprises:
- alternately illuminating eggs in said first group of grid positions and eggs in said second group of grid positions.

11. Method according to any of the claims 9 - 10, wherein said conveyer unit (5, 13), said image recording unit (2, 3) and said illumination unit (4, 52) are mutually arranged such that each egg is illuminated between four times and ten times, preferably between four times and six times, even more preferably four times, and such that each time said egg is illuminated an image is recorded, and such that for each recorded image said egg is rotated between 80 degrees and 120 degrees, preferably between 80 and 100 degrees, even more preferably around 90 degrees.

12. Method according to any of the claims 9 - 11, wherein said inspection device further comprises:
- an egg detection unit (12a-12f) arranged for detecting whether eggs are received in said receiving holders,
and wherein said illumination unit (4, 52) is further arranged to refrain from illuminating a receiving holder in case said egg detection unit has not detected a received egg in said receiving holder, wherein said method further comprises the steps of:
- detecting, by said egg detection unit (12a-12f), whether eggs are received in said receiving holders, and
- refraining, by said illumination unit (4, 52), from illuminating a receiving holder in case said egg detection unit has not detected a received egg in said receiving holder.

13. Method according to any of the claims 9 - 12, wherein said inspection device (1) further comprises:
- an egg size detection unit arranged for detecting sizes of said eggs in said receiving holders,
and wherein said illumination unit (4, 52) is further arranged for determining said intensity of said illumination based on said detected sizes of said eggs, wherein said method further comprises the steps of:
- detecting, by said egg size detection unit, said sizes of said eggs in said receiving holders, and
- determining, by said illumination unit (4, 52), said intensity of said illumination based on said detected sizes of said eggs.

14. Method according to any of the claims 9 - 13, wherein said step of illuminating further comprises:
- illuminating, by said illumination unit (4, 52), eggs in five rows of said uniform grid, wherein a direction of a row is transverse to said conveying direction, and eggs in four columns of said uniform grid, wherein a direction of a column is parallel to said conveying direction, or to
- illuminating, by said illumination unit (4, 52), eggs in six rows of said uniform grid and eggs in four columns of said uniform grid.

15. Computer program product, comprising a readable storage medium, comprising instructions which, when executed on at least one processor, cause the inspection device according to any of the claims 1 - 8 to carry out the method according to any of the claims 9 - 14.

## Patentansprüche

1. Inspektionsvorrichtung (1) zum Inspizieren von Eiern auf Unregelmäßigkeiten, wobei die Inspektionsvorrichtung (1) Folgendes umfasst:
- eine Fördereinheit (5, 13), die Eieraufnahmehalter (17) umfasst, wobei die Aufnahmehalter (17) so platziert sind, dass sie ein gleichmäßiges Gitter von Aufnahmehaltern bilden, wobei die Fördereinheit (5, 13) zum Befördern von in den Aufnahmehaltern (17) aufgenommenen Eiern in einer Förderrichtung angeordnet ist,
- eine Bildaufzeichnungseinheit (2, 3), die bei der Nutzung über der Fördereinheit (5) positioniert und zum Aufzeichnen von Bildern der Eier während der Beförderung angeordnet ist, wobei jedes Bild Eier in einem jeweiligen gleichmäßigen Gitter von Aufnahmehaltern (17) umfasst;
- eine Beleuchtungseinheit (4, 52), die bei der Nutzung unter den Eieraufnahmehaltern (17) positioniert und zum Beleuchten der Eier in dem jeweiligen gleichmäßigen Gitter von Aufnahmehaltern (17) angeordnet ist, wobei die Beleuchtungseinheit (52) eine Vielzahl von Lichtquellen (53a-53d, 54a, 55a, 56a, 57a) umfasst, die je dazu angeordnet sind, ein Ei in dem jeweiligen gleichmäßigen Gitter von Aufnahmehaltern (17, 59) zu beleuchten,
- eine Datenverarbeitungseinheit (7), die zum Bestimmen, ob Eier Unregelmäßigkeiten aufweisen, basierend auf den aufgezeichneten Bildern angeordnet ist;
**dadurch gekennzeichnet, dass**
die Fördereinheit zum Drehen der Eier in den Aufnahmehaltern während der Beförderung angeordnet ist, wodurch ermöglicht wird, dass die Bildaufzeichnungseinheit mehrere Bilder jedes der Eier aufzeichnet, wobei für jedes Bild das Ei mit Bezug auf die vorherigen Bilder gedreht wird;
die Beleuchtungseinheit (4, 52) zum Beleuchten der Eier in dem jeweiligen gleichmäßigen Gitter von Aufnahmehaltern (17) angeordnet ist, sodass nebeneinander befindliche, in der Förderrichtung betrachtete Eier in dem jeweiligen gleichmäßigen Gitter mit unterschiedlicher Intensität beleuchtet werden, oder
die Beleuchtungseinheit (4, 52) zum Beleuchten von Eiern in dem jeweiligen gleichmäßigen Gitter von Aufnahmehaltern (17) angeordnet ist, sodass nebeneinander befindliche, in der Förderrichtung betrachtete Eier in dem jeweiligen gleichmäßigen Gitter abwechselnd beleuchtet werden.

2. Inspektionsvorrichtung (1) nach Anspruch 1, wobei das jeweilige gleichmäßige Gitter einem Schachbrett (101, 102) ähnelt, wobei eine erste Gruppe von Gitterpositionen in dem jeweiligen Gitter weißen Feldern des Schachbretts ähnelt und wobei eine zweite Gruppe von Gitterpositionen in dem jeweiligen Gitter schwarzen Feldern des Schachbretts ähnelt, wobei die Beleuchtungseinheit (4, 52) ferner für Folgendes angeordnet ist:
- abwechselndes Beleuchten von Eiern in der ersten Gruppe von Gitterpositionen und von Eiern in der zweiten Gruppe von Gitterpositionen.

3. Inspektionsvorrichtung (1) nach einem der vorherigen Ansprüche, wobei die Fördereinheit (5, 13), die Bildaufzeichnungseinheit (2, 3) und die Beleuchtungseinheit (4, 52) so zueinander angeordnet sind, dass jedes Ei zwischen vier- und zehnmal, bevorzugter zwischen vier- und sechsmal, noch bevorzugter viermal beleuchtet wird und dass jedes Mal, wenn das Ei beleuchtet wird, ein Bild aufgezeichnet wird und dass für jedes aufgezeichnete Bild das Ei um zwischen 80 und 120 Grad, bevorzugter um zwischen 80 und 100 Grad, noch bevorzugter um ungefähr 90 Grad gedreht wird.

4. Inspektionsvorrichtung (1) nach einem der vorherigen Ansprüche, wobei die Inspektionsvorrichtung ferner Folgendes umfasst:
- eine Eierdetektionseinheit (12a-12f), die zum Detektieren, ob Eier in den Aufnahmehaltern aufgenommen werden, angeordnet ist,
und wobei die Beleuchtungseinheit (4, 52) ferner dazu angeordnet ist, einen Aufnahmehalter nicht zu beleuchten, wenn die Eierdetektionseinheit kein aufgenommenes Ei in dem Aufnahmehalter detektiert hat.

5. Inspektionsvorrichtung (1) nach einem der vorherigen Ansprüche, wobei die Inspektionsvorrichtung (1) ferner Folgendes umfasst:
- eine Eiergrößendetektionseinheit, die zum Detektieren von Größen der Eier in den Aufnahmehaltern angeordnet ist,
und wobei die Beleuchtungseinheit (4, 52) ferner zum Bestimmen der Intensität der Beleuchtung basierend auf den detektierten Größen der Eier angeordnet ist.

6. Inspektionsvorrichtung (1) nach einem der vorherigen Ansprüche, wobei die Beleuchtungseinheit (4, 52) für Folgendes angeordnet ist:
- Beleuchten von Eiern in fünf Zeilen des gleichmäßigen Gitters, wobei eine Richtung einer Zeile parallel zu der Förderrichtung (16) verläuft, und von Eiern in vier Spalten des gleichmäßigen Gitters, wobei eine Richtung einer Spalte quer zu der Förderrichtung (16) verläuft, oder
- Beleuchten von Eiern in sechs oder sieben Zeilen des gleichmäßigen Gitters und von Eiern in vier Spalten des gleichmäßigen Gitters.

7. Inspektionsvorrichtung (1) nach einem der vorherigen Ansprüche, wobei die Beleuchtungseinheit eine Vielzahl von Leuchtdioden, LEDs (53a-53d, 54a, 55a, 56a, 57a), umfasst, wobei jede LED (53a-53d, 54a, 55a, 56a, 57a) dazu angeordnet ist, ein Ei an einer Gitterposition des jeweiligen gleichmäßigen Gitters zu beleuchten.

8. Inspektionsvorrichtung (1) nach Anspruch 7, wobei jede der Vielzahl von LEDs (53a-53d, 54a, 55a, 56a, 57a) mit Richteinrichtungen zum Richten der Beleuchtung zu dem entsprechenden Ei an der Gitterposition des jeweiligen gleichmäßigen Gitters hin versehen ist.

9. Verfahren zum Bestimmen, ob Eier Unregelmäßigkeiten aufweisen, unter Verwendung einer Inspektionsvorrichtung (1) nach einem der vorherigen Ansprüche, wobei das Verfahren die folgenden Schritte umfasst:
- Befördern, durch die Fördereinheit (5, 13), von in den Aufnahmehaltern aufgenommenen Eiern und
- Aufzeichnen, durch die Bildaufzeichnungseinheit (2, 3), von Bildern der Eier während der Beförderung, wobei jedes Bild Eier in einem jeweiligen gleichmäßigen Gitter von Aufnahmehaltern umfasst;
- Beleuchten, durch die Beleuchtungseinheit (4, 52), der Eier in dem jeweiligen gleichmäßigen Gitter von Aufnahmehaltern und
- Bestimmen, durch die Datenverarbeitungseinheit (7), ob Eier Unregelmäßigkeiten aufweisen, basierend auf den aufgezeichneten Bildern,
**dadurch gekennzeichnet, dass** es ferner den folgenden Schritt umfasst:
Drehen, durch die Fördereinheit (5, 13), der Eier in den Aufnahmehaltern während der Beförderung;
wobei der Schritt des Aufzeichnens ferner Folgendes umfasst:
Aufzeichnen, durch die Bildaufzeichnungseinheit (2, 3), mehrerer Bilder jedes der Eier, wobei für jedes Bild das Ei mit Bezug auf die vorherigen Bilder gedreht wird;
wobei der Schritt des Beleuchtens ferner Folgendes umfasst: Beleuchten, durch die Beleuchtungseinheit (4, 52), der Eier in dem jeweiligen gleichmäßigen Gitter von Aufnahmehaltern, sodass nebeneinander befindliche, in der Förderrichtung betrachtete Eier in dem jeweiligen gleichmäßigen Gitter mit unterschiedlicher Intensität beleuchtet werden; oder
Beleuchten, durch die Beleuchtungseinheit (4, 52), der Eier in dem jeweiligen gleichmäßigen Gitter von Aufnahmehaltern, sodass nebeneinander befindliche, in der Förderrichtung betrachtete Eier in dem jeweiligen gleichmäßigen Gitter abwechselnd beleuchtet werden.

10. Verfahren nach Anspruch 9, wobei das jeweilige gleichmäßige Gitter einem Schachbrett (101, 102) ähnelt, wobei eine erste Gruppe von Gitterpositionen in dem jeweiligen Gitter weißen Feldern des Schachbretts ähnelt und wobei eine zweite Gruppe von Gitterpositionen in dem jeweiligen Gitter schwarzen Feldern des Schachbretts ähnelt, wobei der Schritt des Beleuchtens ferner Folgendes umfasst:
- abwechselndes Beleuchten von Eiern in der ersten Gruppe von Gitterpositionen und von Eiern in der zweiten Gruppe von Gitterpositionen.

11. Verfahren nach einem der Ansprüche 9-10, wobei die Fördereinheit (5, 13), die Bildaufzeichnungseinheit (2, 3) und die Beleuchtungseinheit (4, 52) so zueinander angeordnet sind, dass jedes Ei zwischen vier- und zehnmal, bevorzugter zwischen vier- und sechsmal, noch bevorzugter viermal beleuchtet wird und dass jedes Mal, wenn das Ei beleuchtet wird, ein Bild aufgezeichnet wird und dass für jedes aufgezeichnete Bild das Ei um zwischen 80 und 120 Grad, bevorzugter um zwischen 80 und 100 Grad, noch bevorzugter um ungefähr 90 Grad gedreht wird.

12. Verfahren nach einem der Ansprüche 9-11, wobei die Inspektionsvorrichtung ferner Folgendes umfasst:
- eine Eierdetektionseinheit (12a-12f), die zum Detektieren, ob Eier in den Aufnahmehaltern aufgenommen werden, angeordnet ist,
und wobei die Beleuchtungseinheit (4, 52) ferner dazu angeordnet ist, einen Aufnahmehalter nicht zu beleuchten, wenn die Eierdetektionseinheit kein aufgenommenes Ei in dem Aufnahmehalter detektiert hat, wobei das Verfahren ferner die folgenden Schritte umfasst:
- Detektieren, durch die Eierdetektionseinheit (12a-12f), ob Eier in den Aufnahmehaltern aufgenommen werden, und
- Nichtbeleuchten, durch die Beleuchtungseinheit (4, 52), eines Aufnahmehalters, wenn die Eierdetektionseinheit kein aufgenommenes Ei in dem Aufnahmehalter detektiert hat.

13. Verfahren nach einem der Ansprüche 9-12, wobei die Inspektionsvorrichtung (1) ferner Folgendes umfasst:
- eine Eiergrößendetektionseinheit, die zum Detektieren von Größen der Eier in den Aufnahmehaltern angeordnet ist,
und wobei die Beleuchtungseinheit (4, 52) ferner zum Bestimmen der Intensität der Beleuchtung basierend auf den detektierten Größen der Eier angeordnet ist, wobei das Verfahren ferner die folgenden Schritte umfasst:
- Detektieren, durch die Eiergrößendetektionseinheit, der Größen der Eier in den Aufnahmehaltern und
- Bestimmen, durch die Beleuchtungseinheit (4, 52), der Intensität der Beleuchtung basierend auf den detektierten Größen der Eier.

14. Verfahren nach einem der Ansprüche 9-13, wobei der Schritt des Beleuchtens ferner Folgendes umfasst:
- Beleuchten, durch die Beleuchtungseinheit (4, 52), von Eiern in fünf Zeilen des gleichmäßigen Gitters, wobei eine Richtung einer Zeile quer zu der Förderrichtung verläuft, und von Eiern in vier Spalten des gleichmäßigen Gitters, wobei eine Richtung einer Spalte parallel zu der Förderrichtung verläuft, oder
- Beleuchten, durch die Beleuchtungseinheit (4, 52), von Eiern in sechs Zeilen des gleichmäßigen Gitters und von Eiern in vier Spalten des gleichmäßigen Gitters.

15. Computerprogrammprodukt, das ein lesbares Speichermedium umfasst, das Anweisungen umfasst, die, wenn sie auf mindestens einem Prozessor ausgeführt werden, bewirken, dass die Inspektionsvorrichtung nach einem der Ansprüche 1-8 das Verfahren nach einem der Ansprüche 9-14 durchführt.

## Revendications

1. Dispositif d'inspection (1) pour détecter des irrégularités dans des œufs, ledit dispositif d'inspection (1) comprenant :
- une unité de transport (5, 13) comprenant des supports de réception d'œufs (17), dans lequel lesdits supports de réception (17) sont situés de sorte qu'ils forment une grille uniforme de supports de réception, dans lequel ladite unité de transport (5, 13) est agencée pour transporter, dans une direction de transport, des œufs reçus dans lesdits supports de réception (17),
- une unité d'enregistrement d'image (2, 3) positionnée, en utilisation, au-dessus de ladite unité de transport (5) et agencée pour enregistrer des images desdits œufs pendant ledit transport, dans lequel chaque image comprend des œufs dans une grille uniforme particulière de supports de réception (17) ;
- une unité d'éclairage (4, 52) positionnée, en utilisation, en dessous desdits supports de réception d'œufs (17) et agencée pour éclairer lesdits œufs dans ladite grille uniforme particulière de supports de réception (17), dans lequel ladite unité d'éclairage (52) comprend une pluralité de sources de lumière (53a-53d, 54a, 55a, 56a, 57a) agencées chacune pour éclairer un œuf dans ladite grille uniforme particulière de supports de réception (17, 59),
- une unité de traitement de données (7) agencée pour déterminer si les œufs présentent des irrégularités sur la base desdites images enregistrées ;
**caractérisé en ce que**
ladite unité de transport est agencée pour faire tourner lesdits œufs dans lesdits supports de réception pendant ledit transport, permettant à ladite unité d'enregistrement d'image d'enregistrer de multiples images de chacun desdits œufs, dans lequel pour chaque image, l'œuf est tourné par rapport aux images précédentes ;
ladite unité d'éclairage (4, 52) est agencée pour éclairer lesdits œufs dans ladite grille uniforme particulière de supports de réception (17) de sorte que des œufs adjacents, vus dans ladite direction de transport, dans ladite grille uniforme particulière soient éclairés à une intensité différente, ou
ladite unité d'éclairage (4, 52) est agencée pour éclairer des œufs dans ladite grille uniforme particulière de supports de réception (17) de sorte que des œufs adjacents, vus dans ladite direction de transport, dans ladite grille uniforme particulière soient éclairés en alternance.

2. Dispositif d'inspection (1) selon la revendication 1, dans lequel ladite grille uniforme particulière ressemble à un damier (101, 102), dans lequel un premier groupe de positions de grille dans ladite grille particulière ressemble aux champs blancs dudit damier et dans lequel un second groupe de positions de grille dans ladite grille particulière ressemble aux champs noirs dudit damier, dans lequel ladite unité d'éclairage (4, 52) est en outre agencée pour :
- éclairer en alternance les œufs dans ledit premier groupe de positions de grille et les œufs dans ledit second groupe de positions de grille.

3. Dispositif d'inspection (1) selon l'une des revendications précédentes, dans lequel ladite unité de transport (5, 13), ladite unité d'enregistrement d'image (2, 3) et ladite unité d'éclairage (4, 52) sont mutuellement agencées de sorte que chaque œuf soit éclairé entre quatre et dix fois, plus préférablement entre quatre et six fois, encore plus préférablement quatre fois, et de sorte que chaque fois que ledit œuf est éclairé une image soit enregistrée, et de sorte que pour chaque image enregistrée ledit œuf soit tourné entre 80 degrés et 120 degrés, de préférence entre 80 et 100 degrés, encore plus préférablement autour de 90 degrés.

4. Dispositif d'inspection (1) selon l'une des revendications précédentes, dans lequel ledit dispositif d'inspection comprend en outre :
- une unité de détection d'œufs (12a-12f) agencée pour détecter si des œufs sont reçus dans lesdits supports de réception,
et dans lequel ladite unité d'éclairage (4, 52) est en outre agencée pour interdire l'éclairage d'un support de réception dans le cas où ladite unité de détection d'œufs n'a pas détecté d'œuf reçu dans ledit support de réception.

5. Dispositif d'inspection (1) selon l'une des revendications précédentes, dans lequel ledit dispositif d'inspection (1) comprend en outre :
- une unité de détection de taille d'œuf agencée pour détecter les tailles desdits œufs dans lesdits supports de réception,
et dans lequel ladite unité d'éclairage (4, 52) est en outre agencée pour déterminer ladite intensité dudit éclairage sur la base desdites tailles détectées desdits œufs.

6. Dispositif d'inspection (1) selon l'une des revendications précédentes, dans lequel ladite unité d'éclairage (4, 52) est agencée pour
- éclairer des œufs dans cinq rangées de ladite grille uniforme, dans lequel une direction d'une rangée est parallèle à ladite direction de transport (16), et des œufs dans quatre colonnes de ladite grille uniforme, dans lequel une direction d'une colonne est transversale à ladite direction de transport (16), ou pour
- éclairer des œufs dans six ou sept rangées de ladite grille uniforme et des œufs dans quatre colonnes de ladite grille uniforme.

7. Dispositif d'inspection (1) selon l'une des revendications précédentes, dans lequel ladite unité d'éclairage comprend une pluralité de diodes électroluminescentes, LED (53a-53d, 54a, 55a, 56a, 57a), dans lequel chaque LED (53a-53d, 54a, 55a, 56a, 57a) est agencée pour éclairer un œuf dans une position de grille de ladite grille uniforme particulière.

8. Dispositif d'inspection (1) selon la revendication 7, dans lequel chacune de ladite pluralité de LED (53a-53d, 54a, 55a, 56a, 57a) est pourvue de moyens de direction pour diriger l'éclairage vers ledit œuf correspondant dans ladite position de grille de ladite grille uniforme particulière.

9. Procédé permettant de déterminer si des œufs présentent des irrégularités en utilisant un dispositif d'inspection (1) selon l'une des revendications précédentes, dans lequel ledit procédé comprend les étapes de :
- transport, par ladite unité de transport (5, 13), d'œufs reçus dans lesdits supports de réception, et,
- enregistrement, par ladite unité d'enregistrement d'image (2, 3), d'images desdits œufs pendant ledit transport, dans lequel chaque image comprend des œufs dans une grille uniforme particulière de supports de réception ;
- éclairage, par ladite unité d'éclairage (4, 52), desdits œufs dans ladite grille uniforme particulière de supports de réception, et
- détermination, par ladite unité de traitement de données (7), consistant à savoir si des œufs présentent des irrégularités sur la base desdites images enregistrées,
**caractérisé en ce qu'**il comprend en outre l'étape de :
rotation, par ladite unité de transport (5, 13), desdits œufs dans lesdits supports de réception pendant ledit transport ;
ladite étape d'enregistrement comprenant en outre :
l'enregistrement, par ladite unité d'enregistrement d'image (2, 3), de multiples images de chacun desdits œufs, dans lequel pour chaque image, l'œuf est tourné par rapport aux images précédentes ;
ladite étape d'éclairage comprend en outre :
l'éclairage, par ladite unité d'éclairage (4, 52), desdits œufs dans ladite grille uniforme particulière de supports de réception de sorte que des œufs adjacents, vus dans ladite direction de transport, dans ladite grille uniforme particulière soient éclairés à une intensité différente ; ou
l'éclairage, par ladite unité d'éclairage (4, 52), desdits œufs dans ladite grille uniforme particulière de supports de réception de sorte que des œufs adjacents, vus dans ladite direction de transport, dans ladite grille uniforme particulière soient éclairés en alternance.

10. Procédé selon la revendication 9, dans lequel ladite grille uniforme particulière ressemble à un damier (101, 102), dans lequel un premier groupe de positions de grille dans ladite grille particulière ressemble aux champs blancs dudit damier et dans lequel un second groupe de positions de grille dans ladite grille particulière ressemble aux champs noirs dudit damier, dans lequel ladite étape d'éclairage comprend en outre :
- l'éclairage alterné d'œufs dans ledit premier groupe de positions de grille et d'œufs dans ledit second groupe de positions de grille.

11. Procédé selon l'une des revendications 9 et 10, dans lequel ladite unité de transport (5, 13), ladite unité d'enregistrement d'image (2, 3) et ladite unité d'éclairage (4, 52) sont mutuellement agencées de sorte que chaque œuf soit éclairé entre quatre fois et dix fois, de préférence entre quatre fois et six fois, encore plus préférablement quatre fois, et de sorte que chaque fois que ledit œuf est éclairé une image soit enregistrée, et de sorte que pour chaque image enregistrée ledit œuf soit tourné entre 80 degrés et 120 degrés, de préférence entre 80 et 100 degrés, encore plus préférablement autour de 90 degrés.

12. Procédé selon l'une des revendications 9 à 11, dans lequel ledit dispositif d'inspection comprend en outre :
- une unité de détection d'œufs (12a-12f) agencée pour détecter si des œufs sont reçus dans lesdits supports de réception,
et dans lequel ladite unité d'éclairage (4, 52) est en outre agencée pour interdire l'éclairage d'un support de réception dans le cas où ladite unité de détection d'œufs n'a pas détecté d'œuf reçu dans ledit support de réception, dans lequel ledit procédé comprend en outre les étapes de :
- détection, par ladite unité de détection d'œufs (12a-12f), si des œufs sont reçus dans lesdits supports de réception, et
- interdiction, par ladite unité d'éclairage (4, 52), d'éclairer un support de réception dans le cas où ladite unité de détection d'œufs n'a pas détecté d'œuf reçu dans ledit support de réception.

13. Procédé selon l'une des revendications 9 à 12, dans lequel ledit dispositif d'inspection (1) comprend en outre :
- une unité de détection de taille d'œuf agencée pour détecter les tailles desdits œufs dans lesdits supports de réception,
et dans lequel ladite unité d'éclairage (4, 52) est en outre agencée pour déterminer ladite intensité dudit éclairage sur la base desdites tailles détectées desdits œufs, dans lequel ledit procédé comprend en outre les étapes de :
- détection, par ladite unité de détection de taille d'œuf, desdites tailles desdits œufs dans lesdits supports de réception, et
- détermination, par ladite unité d'éclairage (4, 52), de ladite intensité dudit éclairage sur la base desdites tailles détectées desdits œufs.

14. Procédé selon l'une des revendications 9 à 13, dans lequel ladite étape d'éclairage comprend en outre :
- l'éclairage, par ladite unité d'éclairage (4, 52), d'œufs dans cinq rangées de ladite grille uniforme, dans lequel une direction d'une rangée est transversale à ladite direction de transport, et d'œufs dans quatre colonnes de ladite grille uniforme, dans lequel une direction d'une colonne est parallèle à ladite direction de transport, ou
- l'éclairage, par ladite unité d'éclairage (4, 52), d'œufs dans six rangées de ladite grille uniforme et d'œufs dans quatre colonnes de ladite grille uniforme.

15. Produit de programme informatique, comprenant un support de stockage lisible, comprenant des instructions qui, lorsqu'elles sont exécutées sur au moins un processeur, amènent le dispositif d'inspection selon l'une des revendications 1 à 8 à réaliser le procédé selon l'une des revendications 9 à 14.
